# EUROPÄISCHE PATENTSCHRIFT

(11) **EP 1 453 523 B1**
(45) Veröffentlichungstag und Bekanntmachung des Hinweises auf die Patenterteilung: **01.03.2006**
(21) Anmeldenummer: 02791625.3
(22) Anmeldetag: 11.12.2002
(51) Int. Cl.: A61K 31/727, A61K 31/728, A61K 31/79, A61K 31/717, A61K 31/78, A61K 45/06, A61K 31/765, A61P 27/02, A61K 31/737

(54) **HEPARIN-HALTIGES OPHTHALMIKUM**
HEPARIN-CONTAINING OPHTHALMIC AGENT
AGENT OPHTALMIQUE CONTENANT DE L'HEPARINE

(30) Priorität: 12.12.2001 DE 10161149
(43) Veröffentlichungstag der Anmeldung: 08.09.2004
(73) Patentinhaber: URSAPHARM Arzneimittel GmbH & Co. KG, 66129 Saarbrücken (DE)
(72) Erfinder: GROSS, Dorothea, 66386 St. Ingbert (DE); HOLZER, Frank, 66386 St. Ingbert (DE)
(74) Vertreter: Walcher, Armin
(86) Internationale Anmeldenummer: PCT/DE2002/004526
(87) Internationale Veröffentlichungsnummer: WO 2003/053452

(56) Entgegenhaltungen:
- EP-A- 0 698 388
- EP-B- 0 590 655
- WO-A-01/74330
- DE-A- 3 440 352
- DE-A- 19 547 972
- US-A- 3 232 833
- CONDON P I ET AL: "DOUBLE BLIND, RANDOMISED, PLACEBO CONTROLLED, CROSSOVER, MULICENTRESTUDY TO DETERMINE THE EFFICACY OF A 0.1% (W/V) SODIUM HYALURONATE SOLUTION (FERMAVISC) IN THE TREATMENT OF DRY EYE SYNDROME" BRITISH JOURNAL OF OPHTHALMOLOGY, LONDON, GB, Bd. 83, Nr. 10, Oktober 1999 (1999-10), Seiten 1121-1124, XP001008246 ISSN: 0007-1161
- ARAGONA P ET AL: "LONG TERM TREATMENT WITH SODIUM HYALURONATE-CONTAINING ARTIFICIAL TEARS REDUCES OCULAR SURFACE DAMAGE IN PATIENTS WITH DRY EYE" BRITISH JOURNAL OF OPHTHALMOLOGY, LONDON, GB, Bd. 86, Nr. 2, Februar 2002 (2002-02), Seiten 181-184, XP001095826 ISSN: 0007-1161
- ANDERSON W ET AL: "Topical heparin inhibits compound 48/80 induced allergic conjunctivitis." INVESTIGATIVE OPHTHALMOLOGY & VISUAL SCIENCE, Bd. 35, Nr. 4, 1994, Seite 1291 XP001147119 Annual Meeting of the Association for Research in Vision and Ophthalmology;Sarasota, Florida, USA; May 1-6, 1994 ISSN: 0146-0404
- OECHSNER M ET AL: "Polyacrylic acid/polyvinylpyrrolidone bipolymeric systems. I. Rheological and mucoadhesive properties of formulations potentially useful for the treatment of dry-eye-syndrome" EUROPEAN JOURNAL OF PHARMACEUTICS AND BIOPHARMACEUTICS, ELSEVIER SCIENCE PUBLISHERS B.V., AMSTERDAM, NL, Bd. 47, Nr. 2, 1. März 1999 (1999-03-01), Seiten 113-118, XP004257050 ISSN: 0939-6411

## Beschreibung

Die Erfindung betrifft die Verwendung von Wirkstoffen zur Herstellung einer pharmazeutischen Zusammensetzung zur Behandlung ophthalmologischer Fehlfunktionen.

Aus der DE 195 47 972 A1 ist die Verwendung von Heparin oder seiner pharmakologisch verträglichen Salze zur Prophylaxe und akuten Therapie der allergischen Konjunktivitis bekannt. Insbesondere wird dabei Heparin in Form von Augentropfen oder eines Augensprays mit oder ohne Adjuvantien oder Konservierungsstoffe verwendet.

Heparin wird weiterhin zur Behandlung von Verätzungen oder Verbrennungen sowie weiterer Verletzungen der Hornhaut des Auges, bei denen eine rasche Wundheilung der Cornea erforderlich ist, verwendet.

Der exakte Wirkmechanismus des Heparins, das in der systemischen Anwendung primär als Antikoagulans eingesetzt wird, ist nicht geklärt. Neuere Forschungsergebnisse weisen auf Wechselwirkungen des Heparins mit epithelialen Wachstumsfaktoren hin (Denk, P.O. et al., Invest. Ophthalmol. Vis. Sci. (1996) 37, 1005, und Knorr, M. et al., Ophthalmologe (1996) 93, 275). Weiterhin wird eine Modulation des Immunsystems und des Entzündungsgeschehens durch das Heparin diskutiert (Bowler, S.D. et al., Am. Rev. Respir. Dis. (1993) 147, 160).

Des weiteren liegen seit einigen Jahren Erkenntnisse über eine antiallergische Wirkung des Heparins vor. Danach inhibiert Heparin die durch Antigene hervorgerufene Degranulation von Mastzellen ohne direkte antihistamine Wirkung. Diese Wirkung konnte sowohl bei inhalativer als auch nach intravenöser Applikation beobachtet werden (Bowler, S.D. et al., Am. Rev. Respir. Dis (1993) 147, 160; Ahmed, T. et al., N. Engl. J. Med. (1993) 329, 2: 90; Lucio, J. et al., J. Appl. Physiol. (1992) 73, 1093). Weiterhin ist aus tierpharmakologischen Experimenten bekannt, daß sich hochdosieries Heparin nach topischer Applikation in das Auge als wirksam zur Behandlung allergisch bedingter konjunktivaler Entzündungen erwies (Anderson, W. et al., Invest. Ophthalmol. Vis. Sci. (1994) 35, 1291).

Die Applikation von Heparin in das Auge eignet sich dazu, eine allergische Überreaktion des Immunsystems zu vermeiden bzw. zu begrenzen. Nach neueren Erkenntnissen sind allergisch bedingte Reizungen des Auges mit dem Krankheitsbild des "Trockenen Auges" assoziiert.

Das Krankheitsbild des "Trockenen Auges" wird auch als Sicca-Syndrom oder auch als Sicca-Symptomatik bezeichnet. Das Krankheitsbild des "Trockenen Auges", dessen Symptome sich unter anderem in Brennen, Kratzen, Sandkorngefühl im Auge und verschwommenen Sehen äußern, ist auf funktionelle Störungen des Tränenfilms zurückzuführen. Als Ursache für diese funktionellen Störungen kommen beispielsweise auch die Allergien hervorrufende Umwelteinflüsse, wie beispielsweise Schadstoff- oder Ozonbelastung, in Frage. Insbesondere die in Sommermonaten ansteigende Ozonbelastung kann nicht nur eine Störung der Tränenproduktion verursachen, sondern auch eine Zerstörung einzelner Bestandteile des Tränenfilms. Beispielsweise werden in natürlichem Tränenfilm enthaltene Proteine und Hyaluronsäure durch Einwirkung von Ozon zerstört. Weiterhin hat sich gezeigt, daß das Sicca-Syndrom häufig mit einer durch Kosmetika am Auge hervorgerufenen Kontaktallergie verbunden ist.

Aus Spektrum Augenheilkunde (1998) 3/4: 174-176 ist bekannt, daß hypoosmolare Natrium-Hyaluronat-Tropfen zur Therapie des Krankheitsbildes des "Trockenen Auges" verwendet werden können. Dabei wurde Natrium-Hyaluronat mit einem Molekulargewicht von 5.000.000 Dalton verwendet.

Weiterhin ist aus Spektrum Augenheilkunde (1995) 9/5: 215-217 bekannt, daß bakteriell synthetisiertes Hyaluronat zur Behandlung des Krankheitsbildes des "Trockenen Auges" verwendet werden kann. Aus Jpn. J. Ophthalmol. (1996) 40: 62-65, ist bekannt, daß die Verbesserung der Tränenfilmstabilität durch Natrium-Hyaluronat-Augentropfen dosisabhängig ist.

Die EP 0 590 655 B1 offenbart eine pharmazeutische Zusammensetzung, die Wasser, ein kationisches Polysaccharid-Polymer, ein anionisches therapeutisches Mittel sowie ein Salz, Borsäure oder einen Zucker umfaßt. In der EP 0 590 655 B 1 wird davon ausgegangen, daß das anionische therapeutische Mittel an das kationische Polymer über elektrostatische Kräfte gebunden ist.

Aufgabe der vorliegenden Erfindung ist es, eine Verwendung von Wirkstoffen zur Herstellung einer pharmazeutischen Zusammensetzung anzugeben, die eine bessere Therapie von allergisch bedingten Entzündungen des Auges erlaubt.

Die Aufgabe wird durch Verwendung von wenigstens einem pharmakologisch verträglichen Viskositätsregler und wenigstens einem Mucopolysaccharid mit Heparin-Aktivität sowie gegebenenfalls zusätzlichen pharmazeutischen Hilfsmitteln zur Herstellung einer pharmazeutischen Zusammensetzung zur Behandlung von trockenen Augen oder zur Behandlung einer allergisch bedingten Reizung des Auges gemäß Anspruch 1 gelöst.

Unter einem "pharmakologisch verträglichen Viskositätsregler" wird im Sinne der Erfindung ein Viskositätsregler verstanden, der aus toxikologischer und pharmakologischer Sicht unbedenklich ist und somit zur Therapie am Menschen, wie beispielsweise dem menschlichen Auge, verwendet werden kann.

Unter einem "Viskositätsregler" wird im Sinne der Erfindung eine Verbindung bzw. eine Zusammensetzung verstanden, mit der beispielsweise nach Einbringung der pharmazeutischen Zusammensetzung in eine wäßrige Lösung eine gewünschte Viskosität eingestellt werden kann. Bevorzugt bewirkt der Viskositätsregler, daß die beispielsweise in flüssiger, gelartiger oder pastöser Form vorliegende pharmazeutische Zusammensetzung ein viskoelastisches Verhalten aufweist. Unter einem viskoelastischen Verhalten wird erfindungsgemäß verstanden, daß sich die Viskosität unter der Einwirkung von Druck-, Zug-, Schub- und/oder Scherspannungen ändert. Gemäß einer bevorzugten Ausführungsform weist die beispielsweise in flüssiger, gelartiger oder pastöser Form vorliegende erfindungsgemäße pharmazeutische Zusammensetzung aufgrund des Viskositätsreglers das Verhalten einer Nichtnewtonschen Flüssigkeit auf.

Unter einem "Mucopolysaccharid mit Heparin-Aktivität" wird jedes Mucopolysaccharid bzw. Glycosaminoglykan verstanden, das eine dem Heparin vergleichbare biologische bzw. physiologische Aktivität aufweist. Beispielsweise ruft ein Mucopolysaccharid mit Heparin-Aktivität bei Mastzellen eine vergleichbare biologische Wirkung hervor, indem zum Beispiel eine durch Antigen hervorgerufene Degranulation von Mastzellen abgeschwächt oder inhibiert wird. Das Mucopolysaccharid mit Heparin-Aktivität kann dabei auch eine größere bzw. geringere Aktivität als das körpereigene Heparin eines Patienten aufweisen. Eine gewünschte biologische bzw. physiologische Aktivität kann durch Einstellung des Gehaltes an Mucopolysaccharid in der erfindungsgemäßen pharmzeutischen Zusammensetzung bewirkt werden.

Unter "pharmazeutischen Hilfsmitteln" werden Lösungsmittel, Lösungsvermittler, Lösungsbeschleuniger, Salzbildner, Salze, Puffersubstanzen, Viskositäts- und Konsistenzbeeinflusser, Gelbildner, Emulgatoren, Solubilisatoren, Benetzer, Spreizmittel, Antioxidantien, Konservierungsmittel, Füll- und Trägerstoffe, etc. verstanden.

Bevorzugt wird die gemäß der erfindungsgemäßen Verwendung hergestellte pharmazeutische Zusammensetzung in Form von Augentropfen, Augenlösungen, Augenwässer, Augensprays, Augensalben oder Augentabletten verwendet.

Zur Herstellung einer Augensalbe kann der pharmakologisch verträgliche Viskositätsregler und das wenigstens eine Mucopolysaccharid mit Heparin-Aktivität beispielsweise in eine Mischung aus dickflüssigem Paraffin und weißem Vaselin eingebracht werden. Weiterhin kann in Salben beispielsweise auch dünnflüssiges Paraffin oder Wollwachs verwendet werden.

Bevorzugt wird die pharmazeutische Zusammensetzung in Form eines Augensprays oder in Form von Augentropfen bereitgestellt. In der Regel wird dabei der Viskositätsregler sowie das Mucopolysaccharid mit Heparin-Aktivität in wäßrigen Lösungen aufgelöst.

Die wäßrigen Lösungen sind dabei gemäß einer bevorzugten Ausführungsform, bezogen auf die Tränenflüssigkeit, isotone Lösungen. Bei isotonen Lösungen liegt die Osmolarität etwa bei 300mOsm/l. Gemäß einer weiteren bevorzugten Ausführungsform ist die erfindungsgemäße pharmazeutische Zusammensetzung hypoosmolar. In diesem Fall kann die Osmolarität beispielsweise etwa 160-180 mOsm/l betragen. Eine hypoosmolare Lösung findet insbesondere dann Anwendung, wenn eine abnorm hohe Osmolarität eines Tränenfilms bei einem Patienten mit trockenen Augen ausgeglichen werden muß.

Zur Isotonisierung der wäßrigen Lösung werden Natriumchlorid, Borsäure, etc. verwendet. Der pH-Wert der wäßrigen Lösung liegt in einem Bereich von pH 6 bis 9, bevorzugt bei pH 7,4 bis 7,7. Zur Einstellung des pH-Werts werden Pufferlösungen wie beispielsweise Phosphatpuffer, Acetatpuffer, Acetat-Boratpuffer, Citratpuffer und Boiatpuffer verwendet.

Erfindungsgemäß wird der Viskositätsregler ausgewählt, aus Hyaluronsäure; Hyaluronat, Chondroitinsulfat, Polyvinylpyrrolidon, Polyvinylalkohol, Polyacrylamid, Polyacrylharze, Polyethylenglykol, Cellulosederivate, nämlich Hydroxyethylcellulose, Hydroxypropylmethylcellulose und/oder Carboxymethylcellulose, sowie Mischungen davon.

Es hat sich gezeigt, daß die vorgenannten Viskositätsregler am Auge reizmindernd wirken und eine Schmierwirkung aufweisen. Die Schmierwirkung verzögert den Abfluß der am Auge aufgebrachten pharmazeutischen Zusammensetzung und verlängert somit den Kontakt mit der Hornhaut des Auges. Somit kann das wenigstens eine Mucopolysaccharid mit Heparin-Aktivität über einen längeren Zeitraum, beispielsweise von wenigstens 30 Minuten bis wenigstens 60 Minuten, auf der Cornea gehalten werden. Das wenigstens eine Mucopolysaccharid mit Heparin-Aktivität kann somit bei einer allergisch bedingten Irritation des Auges zuverlässig über einen langen Zeitraum auf das Auge einwirken und den Entzündungsvorgang therapieren.

Durch die viskoelastischen Eigenschaften des pharmakologisch verträglichen Viskositätsreglers bedingt, insbesondere bei Verwendung von Hyaluronsäure oder Hyaluronat als Viskositätsregler, wird das Mucopolysaccharid mit Heparin-Aktivität bei jedem Lidschlag des Auges ohne weiteres wieder im wesentlichen gleichmäßig auf der gesamten Augenoberfläche verteilt, sofern es zu einem gewissen Abfluß gekommen sein sollte.

Die Symptomatik einer allergisch bedingten Irritation des Auges ist mit dem Sicca-Syndrom, d.h. dem Krankheitsbild des "Trockenen Auges", eng vergesellschaftet. Bei dem Sicca-Syndrom kommt es zu einer unzureichenden Tränenfilmbildung. Sowohl bei einer allergischen Reizung des Auges als auch bei dem Sicca-Syndrom kommt es zu Bindehautrötung, Lidschwellung und Juckreiz, d.h. zu vergleichbaren Entzündungssymptomen.

Die bei der erfindungsgemäßen Verwendung hergestellte pharmazeutische Zusammensetzung verringert bzw. verhindert die Freisetzung oder die Wirkung von Histamin sowie von weiteren Allergiemediatoren. Das wenigstens eine Mucopolysaccharid mit Heparin-Aktivität wirkt somit beispielsweise antiallergisch und juckreizstillend. Die pharmazeutische Zusammensetzung mit einer durch den Viskositätsregler eingestellten Viskosität wirkt weiterhin als Gleit- bzw. Schmiermittel und stellt somit einen Ersatz für den beim Sicca-Syndrom auftretenden unzureichenden Tränenfilm dar. Der durch die mechanische Reizung beim Sicca-Syndrom auftretende Juckreiz sowie die Rötung des Auges wird durch die Gleit- bzw. Schmierwirkung bei der gemäß der erfindungsgemäßen Verwendung hergestellten pharmazeutischen Zusammensetzung zuverlässig verringert.

Die gleichzeitige Einwirkung von Mucopolysaccharid mit Heparin-Aktivität sowie die Bereitstellung eines künstlichen Tränenfilms führt zu einer synergistischen Wirkung, die eine raschere Therapie einer allergisch bedingten Irritation des Auges erlaubt. Da das Auge für den Menschen eines der wichtigsten Sinnesorgane ist, stellt die gemäß der erfindungsgemäßen Verwendung hergestellte pharmazeutische Zusammensetzung einen bedeutenden Fortschritt auf dem Gebiet der Ophthalmologie dar.

Gemäß einer weiteren bevorzugten Ausführungsform beträgt die Menge an Hyaluronsäure und/oder die Menge an Hyaluronat 0,005 Gew.-% bis 5 Gew.-%, bevorzugt 0,01 Gew.-% bis 1 Gew.-%, jeweils bezogen auf das Gesamtgewicht der pharmazeutischen Zusammensetzung.

Bevorzugt wird bei der bei der erfindungsgemäßen Verwendung hergestellten pharmazeutischen Zusammensetzung als pharmakologisch verträglicher Viskositätsregler Hyaluronsäure und/oder Hyaluronat verwendet.

Hyaluronsäure ist Bestandteil des Glaskörpers des Auges und stellt insofern keine für den menschlichen Organismus fremde Verbindung dar. Aus diesem Grund ist Hyaluronsäure aus immunologischer Sicht sehr gut verträglich. Darüber hinaus weist Hyaluronsäure bzw. Hyaluronat eine strukturelle Ähnlichkeit mit Mucin auf. Mucin bildet die unterste Schicht des dreischichtigen Tränenfilms und sorgt für eine optimale Benetzung der Hornhaut- und Bindehautepithelien.

Weiterhin weist Hyaluronsäure eine für die Anwendung am Auge hervorragende Eigenschaft auf, daß nämlich die Viskosität mit zunehmender Schergeschwindigkeit ansteigt.

Nach Aufbringung der gemäß der erfindungsgemäßen Verwendung hergestellten pharmazeutischen Zusammensetzung auf die Hornhaut des Auges wird über den Lidschlag des Augenlides eine Scherspannung an die pharmazeutische Zusammensetzung angelegt, wodurch die zunächst erhöhte Viskosität erniedrigt wird. Durch den Lidschlag des Augenlides erniedrigt sich die Viskosität, so daß sich ein gleichmäßiger Film auf der Oberfläche des Auges ausbildet. Nach dem Lidschlag erhöht sich die Viskosität, so daß der Film an der Augenoberfläche gut anhaftet.

Hyaluronsäure bzw. deren Salze, die Hyaluronate, insbesondere Natriumhyaluronat, weist bzw. weisen hervorragende optische Eigenschaften auf, so daß es zu keiner Beeinträchtigung des Visus bei den behandelten Patienten kommt.

Die Verwendung von Hyaluronsäure bzw. Hyaluronaten in der gemäß der erfindungsgemäßen Verwendung hergestellten pharmazeutischen Zusammensetzung ist insbesondere bei Benetzungsstörungen des Auges, d.h. bei dem sogenannten "trockenen Auge" und zur Behandlung von Epithelläsionen, die aus den Benetzungsstörungen resultieren, äußerst vorteilhaft.

Bei Verwendung von Hyaluronsäure und/oder Hyaluronat in der pharmazeutischen Zusammensetzung wird die pharmazeutische Zusammensetzung bevorzugt konservierungsmittelfrei bereitgestellt.

Konservierungsmittel können den präcornealen Tränenfilm schädigen und zu einer Reduzierung der Anzahl der Mikrovilli und Mikroplicae der oberflächlichen Hornhautepitelzellen führen. Insbesondere besitzt das weit verbreitete Benzalkoniumchlorid eine große Schädigungspotenz. Im Hinblick auf die gewünschte Therapie einer allergisch bedingten oder durch das Sicca-Syndrom bedingten Reizung des Auges ist jede weitere Reizung und/oder Schädigung des Auges durch den Zusatz von Konservierungsmittel zu vermeiden.

Bevorzugt wird zur Lagerung und Abgabe einer gemäß der erfindungsgemäßen Verwendung hergestellten konservierungsmittelfreien pharmazeutischen Zusammensetzung das in "PTA heute" 1996, Nr. 12, S. 1230-1232 beschriebene Comod®-System, verwendet, das eine sterile Lagerung und Mehrfachabgabe der angegebenen pharmazeutischen Zusammensetzung erlaubt. Selbstverständlich können auch herkömmliche Einzeldosisbehältnisse verwendet werden, die nach Gebrauch weggeworfen werden.

Besonders bevorzugt beträgt die Menge an Hyaluronsäure und/oder die Menge an Hyaluronat 0,05 Gew.-% bis 0,5 Gew.-%.

Äußerst vorteilhaft besitzt Hyaluronsäure bzw. besitzen Hyaluronate die Eigenschaft, Wasser zu binden. Diese Eigenschaft, Wasser zu binden, ist insbesondere bei der Behandlung des Sicca-Syndroms vorteilhaft, da der unerwünschten Austrocknung der Hornhaut des Auges entgegengewirkt wird. Konzentrationen von 0,1 Gew.-% bis 0,3 Gew.-% haben sich als sehr geeignet erwiesen.

Ein weiterer diagnostischer Parameter bei der Diagnose des Krankheitsbildes des "Trockenen Auges" ist die Tränenfilmaufreißzeit, die eine Aussage über die Qualität der Tränenflüssigkeit erlaubt. Dabei wird beispielsweise der Tränenfilm mit Fluorescein angefärbt und nachfolgend der Patient gebeten, die Augen möglichst lange ohne Blinkreflex offen zu halten. Unter Verwendung einer Spaltlampe wird dann festgestellt, wann der Tränenfilm zum ersten mal aufreißt. Liegt der Zeitraum unter 10 Sekunden, besteht der Verdacht auf das Krankheitsbild des "Trockenen Auges". Dabei hat sich Hyaluronsäure in einer Konzentration von 0,1 Gew.-% bis 0,3 Gew.-% im Hinblick auf die Verlängerung der Tränenfilmaufreißzeit als sehr wirksam erwiesen.

Die Hyaluronsäure bzw. das Hyaluronat kann aus dem Glaskörper des Rinderauges oder aber auch aus Hahnenkämmen isoliert werden. Weiterhin kann Hyaluronsäure bzw. Hyaluronat auch in Bakterienstämmen in pharmazeutischer Qualität hergestellt werden.

Als Salze der Hyaluronsäure können beispielsweise Kalium-, Natrium-, Calcium- und/oder Magnesium-Hyaluronate verwendet werden.

Besonders bevorzugt ist das Hyaluronat Natrium-Hyaluronat.

Wässrige Natriumhyaluronatlösungen sind Flüssigkeiten mit nicht-newtonschen Fließeigenschaften. Aufgrund dieser physikalischen Eigenschaft eignen sich wässrige Natriumhyaluronatlösungen hervorragend als Gleit- und Schmiermittel mit guter Haftwirkung und verlängerter Verweilzeit auf den konjunktivalen und cornealen Epithelien ohne Beeinträchtigung der Sehleistung. Eine Konzentration von 0,1 Gew.-% von Natriumhyaluronat in der gemäß der erfindungsgemäßen Verwendung hergestellten Zusammensetzung verbessert erheblich das bei der Behandlung von trockenen Augen wichtige subjektive Empfinden der Patienten.

Weiterhin zeigen Natriumhyaluronat-haltige Augentropfen im Tierversuch wundheilungsfördernde Eigenschaften auf die Epithelien des Auges. Es wurde festgestellt, daß Hyaluronsäure bzw. Natriumhyaluronat konzentrationsabhängig die Migration von Epithelzellen und somit die Wundheilung fördert. Eine 0,1 Gew.-%ige Natriumhyaluronat-Lösung bewirkte bei Comeaepithelzellen von Kaninchen eine gesteigerte Epithelzellmigration.

Auch bewirkt Hyaluronsäure oder Natriumhyaluronat bei einer Verletzung des Comeaepithels oder bei einer Verätzung der Cornea eine raschere und bessere, d.h. unter weniger Narbenbildung ablaufende Wundheilung.

Der genaue Wirkmechanismus der Wundheilunäsförderung durch Hyaluronsäure ist noch ungeklärt. Während eine Beeinflussung der Durchblutung der umgebenden Zellen als weniger wahrscheinlich erscheint, gibt es verschiedene Hinweise auf eine Wirkung auf Zellen, die im Entzündungsgeschehen eine Rolle spielen.

Schließlich zeigt Hyaluronsäure dosisabhängig eine schützende Wirkung vor Schädigung von Zellen durch Sauerstoffradikale. Freie Sauerstoffradikale verlangsamen die Wundheilung und spielen somit eine entscheidende Rolle im Entzündungsgeschehen.

Die antientzündliche Wirkung von Hyaluronsäure bzw. Hyaluronat sowie der durch Hyaluronsäure bzw. Hyaluronat vermittelte Schutz vor der schädlichen Wirkung von Sauerstoffradikalen wirken in synergistischer Weise mit der anlientzündlichen Wirkung des Mucopolysaccharids mit Heparin-Aktivität in der gemäß der erfindungsgemäßen Verwendung hergestellten pharmazeutischen Zusammensetzung zusammen.

Die weiterhin durch Hyaluronsäure bzw. Hyaluronat in zubereiteten Lösungen, Gelen, Pasten oder Salben bewirkten nicht-newtonschen Fließeigenschaften der pharmazeutischen Zusammensetzung bewirken neben einer hervorrragenden Gleit- und Schmierwirkung auch eine hervorragende Haftung an der Hornhaut des Auges. Die bei dem Sicca-Syndrom auftretende mechanische Reizung des Auges wird stark verringert bzw. beseitigt. Darüber hinaus wird durch die aufgrund der anti-newtonschen Fließeigenschaften verbesserten Haftung der pharmazeutischen Zusammensetzung auf der Hornhaut des Auges eine raschere Heilung der entzündlichen Vorgänge bewirkt.

Gemäß einer weiter bevorzugten Ausführungsform weist die Hyaluronsäure und/oder das Hyaluronat ein Molekulargewicht auf, das in einem Bereich von etwa 50.000 bis etwa 10.000.000 Dalton, bevorzugt von etwa 250.000 bis etwa 5.000.000 Dalton, liegt. Besonders bevorzugt beträgt das Molekulargewicht der Hyaluronsäure bzw. des Hyaluronats 500.000 bis 4.000.000. Dalton. Äußerst bevorzugt weist die Hyaluronsäure bzw. das Hyaluronat ein Molekulargewicht von etwa 1.500.000 bis 3.500.000 Dalton auf.

Das hohe Molekulargewicht der Hyaluronsäure bzw. des verwendeten Hyaluronats wie beispielsweise Natriumhyaluronat bewirkt eine hohe Viskoelastizität bei niedriger Konzentration. In der Lösung liegen die Molekülketten in zufälliger Anordnung knäuelartig vor. Unter dem Einfluß der durch die Bewegung des Augenlides ausgeübten Scherkräfte richten sich die Makromoleküle in etwa parallel aus. Diese Änderung in der dreidimensionalen Struktur unter dem Einfluß von Scherkräften dürfte maßgeblich für die hervorragenden viskoelastischen Eigenschaften sein.

Bei Lidöffnung überzieht die Substanz die Homhautoberfläche und stellt aufgrund der hohen Wasserbindungskapazität von Hyaluronat auch einen Schutz gegen Verdunstung dar. Dies ist insbesondere beim Krankheitsbild des trockenen Auges von Vorteil, bei der es zu einer Verringerung der Menge an Tränenflüssigkeit im Auge kommt.

Weiterhirt ist bevorzugt, daß die Heparin-Aktivität des Mucopolysaccharids in einem Bereich von 200 I.E./ml bis 100.000 I.E./ml, bevorzugt 1.000 I.E./ml bis 50.000 I.E./ml liegt. Bevorzugt liegt die Heparin-Aktivität in einem Bereich von 1.200 I.E./ml bis 10.000 I.E./ml. Sehr bevorzugt liegt die Heparin-Aktivität in einem Bereich von 1.300 I.E./ml bis 5.000 I.E./ml.

Die Heparin-Aktivität wird dabei gemäß Europäischem Arzneibuch 1997 (Pharmacopoea Europaea, 1997) bestimmt. Die Einheit "I.E./ml" ist eine Abkürzung für "Internationale Einheit/ml".

Die Heparin-Aktivität des Mucopolysaccharids kann über jeden geeigneten Test zur Bestimmung der Heparinaktivität bestimmt werden. Wesentlich ist, daß der Vergleich der Aktivität von humanem Heparin und der Hepatinaktivität des Mucopolysaccharids mit dem gleichen Test unter vergleichbaren Testbedingungen durchgeführt wird.

Erfindungsgemäß wird das Mucopolysaccharid mit Heparin-Aktivität ausgewählt aus Heparinoiden, humanem Heparin, tierischem Heparin, rekombinantem Heparin, chemisch modifiziertem Heparin, enzymatisch modifiziertem Heparin, trunkiertem Heparin, niedermolekularem Heparin, Heparansulfat und Mischungen davon.

Das Molekulargewicht von Heparin liegt üblicherweise in einem Bereich von 5.000 bis 30.000 g/mol, bevorzugt 6.000 bis 20.000 g/mol.

Niedermolekulares Heparin weist in der Regel ein Molekulargewicht in einem Bereich von 4.000 bis 8.000 g/mol auf und kann aus natürlichem Heparin durch Depolymerisation, beispielsweise unter Verwendung von Salpetersäure, gewonnen werden.

Sämtliche der vorgenannten Mucopolysaccharide mit Heparin-Aktivität können selbstverständlich auch in Form der jeweiligen pharmakologisch verträglichen Salze bei der gemäß der erfindungsgemäßen Verwendung hergestellten pharmazeutischen Zusammensetzung verwendet werden.

Die genaue Dosierung der Heparin-Aktivität in der pharmazeutischen Zusammensetzung variiert in Abhängigkeit von der zu behandelnden allergisch induzierten Reizung des Auges.

Weiterhin ist bevorzugt, daß die pharmazeutische Zusammensetzung in Form einer Lösung, Suspension, Emulsion, eines Gels, einer Salbe, Paste, eines Pulvers, Granulats oder einer Tablette vorliegt. Die pharmazeutische Zusammensetzung ist bevorzugt ein Ophthalmikum, weiter bevorzugt ein Heparin-haltiges Ophthalmiküm.

Gemäß einer bevorzugten Ausführungsform liegt die pharmazeutische Zusammensetzung in der Form einer Lösung vor, so daß diese beispielsweise in der Form von Augentropfen oder eines Augensprays auf die Hornhautoberfläche des Auges aufgebracht werden kann.

Selbstverständlich ist es möglich, daß die gemäß der erfindungsgemäßen Verwendung hergestellte pharmazeutische Zusammensetzung in der Form eines Feststoffes vorliegt, der vor einer Applikation zunächst in einer wäßrigen Lösung wie beispielsweise einer Pufferlösung aufgelöst wird. Nach Auflösung eines Feststoffes, beispielsweise in einer wäßrigen Pufferlösung, wird diese Lösung sterilfiltriert und kann dann als Augenspray bzw. Augentropfen auf die Cornea aufgebracht werden. Bevorzugt liegen Feststoff und Lösungsmittel bei getrennter Aufbewahrung bereits in steriler Form vor, so daß ein Sterilfiltrieren nach Herstellung der Lösung nicht erforderlich ist. Der Anwender kann somit nach Herstellung der Mischung bzw. Lösung die pharmzeutische Zusammensetzung direkt applizieren.

Bei Bereitstellung der pharmazeutischen Zusammensetzung in Form eines Feststoffs wie beispielsweise eines Pulvers, Puders, Granulats oder einer Tablette umfaßt die gemäß der erfindungsgemäßen Verwendung hergestellte pharmazeutische Zusammensetzung bevorzugt Heparin-Natrium und/oder Heparin-Kalzium sowie Hyaluronsäure und/oder Natriumhyaluronat, da diese Verbindungen in Wasser sehr gut löslich sind. Vor der Applikation werden dann beispielsweise Heparin-Natrium und Hyaluronsäure in den gewünschten Mengenverhältnissen miteinander vermengt und unter Zugabe von Wasser bzw. wäßrigen Pufferlösungen aufgelöst und nachfolgend sterilfiltriert.

Grundsätzlich kann die gemäß der erfindungsgemäßen Verwendung hergestellte pharmazeutische Zusammensetzung auch in Form von Augentabletten in den Bindehautsack eingebracht werden. Unter Einwirkung von Tränenflüssigkeit löst sich die Augentablette rasch auf.

Bevorzugt ist jedoch die Applikation der pharmazeutischen Zusammensetzung in der Form von Augentropfen bzw. Augensprays.

Bei Bereitstellung der pharmazeutischen Zusammensetzung in Form von Augensalben bzw. Augengelen werden die Wirkstoffe beispielsweise in Vaselin oder Paraffin mit und ohne Emulgatorzusatz wie beispielsweise Cholesterin, Wollwachs, Wollwachsalkohole, Cetanol, etc. bereitgestellt.

Besonders bevorzugt wird die gemäß der erfindungsgemäßen Verwendung nach einem der Ansprüche 1 bis 6 hergestellte pharmazeutische Zusammensetzung bei der Behandlung von ophthalmologischen Fehlfunktionen, ausgewählt aus Sicca-Syndrom, allergischer Rhinokonjunktivitis, atopischer Keratokonjunktivitis, allergischer Keratokonjunktivitis, gigantopapillärer Konjunktivitis, Konjunktivitis vernalis, Episkleritis, wie beispielsweise Episcleritis periodica, Episcleritis partialis fugax, Skleritis, Tenonitis, Sjögren-Syndrom und Mischformen davon, verwendet.

### Beispiel 1

2000 I.E. Heparin-Natrium
1,0 mg Natrium-Hyaluronat, Molekulargewicht: 1,5 x 10⁶-3,5 x 10⁶ Dalton
2,0 mg Natriumdihydrogenphosphat-Dihydrat
12,0 mg Dinatriumhydrogenphosphat-Dodecahydrat
27,0 mg Sorbit
ad 1,0 ml Wasser für Injektionszwecke

### Beispiel 2

4000 I.E. Heparin-Natrium,
1,0 mg Natrium-Hyaluronat, Molekulargewicht: 1,5 x 10⁶-3,5 x 10⁶ Dalton
2,0 mg Natriumdihydrogenphosphat
12,0 mg Dinatriumhydrogenphosphat-Dodecahydrat
27,0 mg Sorbit
ad 1,0 ml Wasser für Injektionszwecke

## Patentansprüche

1. Verwendung von wenigstens einem pharmakologisch verträglichen Viskositätsregler und wenigstens einem Mucopolysaccharid mit Heparin-Aktivität sowie gegebenenfalls zusätzlichen pharmazeutischen Hilfsmitteln zur Herstellung einer pharmazeutischen Zusammensetzung zur Behandlung von trockenen Augen oder zur Behandlung einer allergisch bedingten Reizung des Auges, wobei die pharmazeutische Zusammensetzung aus wenigstens einem pharmakologisch verträglichen Viskositätsregler, ausgewählt aus Hyaluronsäure, Hyaluronat, Chondroitinsulfat, Polyvinylpyrrolidon, Polyvinylalkohol, Polyacrylamid, Polyacrylharze, Polyethylenglykol, Cellulosederivate, nämlich Hydroxyethylcellulose, Hydroxypropylmethylcellulose und/oder Carboxymethylcellulose, sowie Mischungen davon, und wenigstens einem Mucopolysaccharid mit Heparinaktivität, das ausgewählt wird aus Heparinoiden, humanem Heparin, tierischem Heparin, rekombinantem Heparin, chemisch modifiziertem Heparin, enzymatisch modifiziertem Heparin, trunkiertem Heparin, niedermolekularem Heparin, Heparansulfat und Mischungen davon, sowie gegebenenfalls zusätzlichen pharmazeutischen Hilfsmittel besteht.

2. Verwendung nach Anspruch 1,
**dadurch gekennzeichnet,**
**daß** die Menge an Hyaluronsäure und/oder die Menge an Hyaluronat 0,005 Gew.-% bis 5 Gew.-%, bevorzugt 0,01 Gew.-% bis 1 Gew.-%, jeweils bezogen auf das Gesamtgewicht der pharmazeutischen Zusammensetzung, beträgt.

3. Verwendung nach Anspruch 1 oder 2,
**dadurch gekennzeichnet,**
**daß** die Hyaluronsäure und/oder das Hyaluronat ein Molekulargewicht aufweist, das in einem Bereich von 50.000 bis 10.000.000 Dalton, bevorzugt von 250.000 bis 5.000.000 Dalton, liegt.

4. Verwendung nach einem der Ansprüche 1 bis 3,
**dadurch gekennzeichnet,**
**daß** das Hyaluronat Natrium-Hyaluronat ist.

5. Verwendung nach einem der Ansprüche 1 bis 4,
**dadurch gekennzeichnet,**
**daß** die Heparin-Aktivität des Mucopolysaccharids in einem Bereich von 500 I.E/ml bis 100.000 I.E./ml, bevorzugt 1.000 I.E./ml bis 50.000 I.E./ml liegt.

6. Verwendung nach einem der Ansprüche 1 bis 5,
**dadurch gekennzeichnet,**
**daß** die pharmazeutische Zusammensetzung in Form einer Lösung, Suspension, Emulsion, eines Gels, einer Salbe, Paste, eines Puders, Pulvers, Granulats oder einer Tablette vorliegt.

7. Verwendung nach einem der Ansprüche 1 bis 6,
**dadurch gekennzeichnet,**
**daß** die trockenen Augen oder die allergisch bedingte Reizung des Auges auf einer ophthalmologischen Fehlfunktion beruhen bzw. beruht, die ausgewählt wird aus Sicca-Syndrom, allergischer Rhinokonjunktivitis, atopischer Keratokonjunktivitis, allergischer Keratokonjunktivitis, gigantopapilläre Konjunktivitis, Konjunktivitis vernalis, Episkleritis, Skleritis, Tenonitis, Sjögren-Syndrom und Mischformen davon.

## Claims

1. Use of at least one pharmacologically tolerable viscosity regulator and at least one mucopolysaccharide having heparin activity, and optionally additional pharmaceutical excipients for the manufacture of a pharmaceutical composition for the treatment of dry eyes or for the treatment of an allergy-related irritation of the eye, the pharmaceutical composition consisting of at least one pharmacologically tolerable viscosity regulator selected from hyaluronic acid, hyaluronate, chondroitin sulphate, polyvinylpyrrolidone, polyvinyl alcohol, polyacrylamide, polyacrylic resins, polyethylene glycol, cellulose derivatives, namely hydroxyethylcellulose, hydroxypropylmethylcellulose and/or carboxymethylcellulose, and mixtures thereof, and at least one mucopolysaccharide having heparin activity, which is selected from heparinoids, human heparin, animal heparin, recombinant heparin, chemically modified heparin, enzymatically modified heparin, truncated heparin, low molecular weight heparin, heparan sulphate and mixtures thereof, and optionally additional pharmaceutical excipients.

2. Use according to Claim 1,
**characterized in that**
the amount of hyaluronic acid and/or the amount of hyaluronate is 0.005% by weight to 5% by weight, preferably 0.01% by weight to 1% by weight, in each case based on the total weight of the pharmaceutical composition.

3. Use according to Claim 1 or 2,
**characterized in that**
the hyaluronic acid and/or the hyaluronate has a molecular weight which lies in a range from 50,000 to 10,000,000 daltons, preferably from 250,000 to 5,000,000 daltons.

4. Use according to one of Claims 1 to 3,
**characterized in that**
the hyaluronate is sodium hyaluronate.

5. Use according to one of Claims 1 to 4,
**characterized in that**
the heparin activity of the mucopolysaccharide lies in a range from 500 IU/ml to 100,000 IU/ml, preferably 1000 IU/ml to 50,000 IU/ml.

6. Use according to one of Claims 1 to 5,
**characterized in that**
the pharmaceutical composition is present in the form of a solution, suspension, emulsion, of a gel, of an ointment, paste, of a dusting powder, oral powder, granules or of a tablet.

7. Use according to one of Claims 1 to 6,
**characterized in that**
the dry eyes or the allergy-related irritation of the eye are or is based on an ophthalmological dysfunction which is selected from sicca syndrome, allergic rhinoconjunctivitis, atopic keratoconjunctivitis, allergic keratoconjunctivitis, gigantopapillary conjunctivitis, vernal conjunctivitis, episcleritis, scleritis, tenonitis, Sjögren's syndrome and mixed forms thereof.

## Revendications

1. Application d'au moins un agent régulateur de viscosité compatible sur le plan pharmacologique et d'au moins un mucopolysaccharide présentant une activité d'héparine ainsi qu'au besoin des moyens pharmaceutiques auxiliaires supplémentaires afin de fabriquer une composition pharmaceutique pour le traitement des yeux secs ou pour le traitement d'une irritation de l'oeil due à une allergie, la composition pharmaceutique se compose d'au moins un agent régulateur de viscosité compatible sur le plan pharmacologique parmi les substances acide hyaluronique, hyaluronate, sulfate de chondroïtine, polyvinylpyrrolidone, alcool polyvinylique, amide polyacrylique, résines polyacryliques, polyéthylèneglycol, dérivés de cellulose à savoir la cellulose hydroxyéthylique, la cellulose hydroxypropylméthylique, et/ou la cellulose carboxyméthylique, ainsi que des mélanges de ces substances, ainsi que d'au moins un mucopolysaccharide présentant une activité d'héparine et sélectionné parmi des héparinoïdes, l'héparine humaine, l'héparine animale, l'héparine recombinante, l'héparine modifiée chimiquement, l'héparine modifiée par des enzymes, l'héparine tronquée, l'héparine de faible poids moléculaire, le sulfate d'héparitine et des mélanges de ces substances, ainsi qu'au besoin des moyens pharmaceutiques auxiliaires supplémentaires.

2. Application selon la revendication 1, **caractérisée en ce que** la quantité d'acide hyaluronique et/ou la quantité d'hyaluronate se situe(nt) entre 0,005% du poids et 5% du poids, de préférence entre 0,01% du poids et 1% du poids, en se référant respectivement au poids total de la composition pharmaceutique.

3. Application selon la revendication 1 ou 2, **caractérisée en ce que** l'acide hyaluronique et/ou l'hyaluronate présente(présentent) un poids moléculaire qui se trouve dans une plage de 50 000 à 10 000 000 dalton, de préférence de 250 000 à 50 000 000 dalton.

4. Application selon l'une des revendications 1 à 3, **caractérisée en ce que** l'hyaluronate est un hyaluronate de sodium.

5. Application selon l'une des revendications 1 à 4, **caractérisée en ce que** l'activité d'héparine du mucopolysaccharide se trouve dans une plage de 500 LE/ml à 100 000 LE/ml, de préférence de 1 000 LE/ml à 50 000 LE/ml.

6. Application selon l'une des revendications 1 à 5, **caractérisée en ce que** la composition pharmaceutique existe sous la forme d'une solution, d'une suspension, d'une émulsion, d'un gel, d'une pommade, d'une pâte, d'une poudre, d'un granulé ou d'un comprimé.

7. Application selon l'une des revendications 1 à 6, **caractérisée en ce que** les yeux secs ou l'irritation de l'oeil due à une allergie reposent/repose sur une fonction ophtalmologique défective qui est sélectionnée parmi les affections que sont le syndrome de Sjögren/Larsson (Sicca), la rhinoconjonctivite allergique, la rhinoconjonctivite atopique, la kératoconjonctivite allergique, la conjonctivite gigantopapillaire, la conjonctivite vénale, l'épisclérite, la sclérite, la ténonite le syndrome de Sjögren et des formes mixtes de ces affections.
